(19) Europäisches Patentamt
European Patent Office
Office européen des brevets

(11) Veröffentlichungsnummer: **0 199 247**
**A2**

(12) # EUROPÄISCHE PATENTANMELDUNG

(21) Anmeldenummer: 86105123.3

(22) Anmeldetag: 14.04.86

(51) Int. Cl.⁴: **A 61 K 31/335**
**C 07 D 303/36**

(30) Priorität: 22.04.85 DE 3514455

(43) Veröffentlichungstag der Anmeldung:
29.10.86 Patentblatt 86/44

(84) Benannte Vertragsstaaten:
AT BE CH DE FR GB IT LI NL SE

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Möller, Hinrich, Dr.**
**Schumannstrasse 11**
**D-4019 Monheim(DE)**

(54) **Arzneimittel mit cytostatischer Wirksamkeit, enthaltend Glycidylamine.**

(57) Arzneimittel mit cytostatischer Wirksamkeit, enthaltend Glycidylamine der allgemeinen Formel

```
      G
     /
R – N          I
     \
      G
```

in der die Reste G und R eine der folgenden Bedeutungen haben:

```
G =          O

     – CH2 – C –CH2

             R1
```

mit $R^1$ = H oder Alkyl mit 1 bis 4 Kolenstoffatomen, R = G oder
Gesättigtes oder ungesättigtes Alkyl oder
Cycloalkyl mit 1 bis 22 Kohlenstoffatomen, wobei die Reste mit einem oder mehreren der folgenden Substituenten substituiert sein können: Halogen, Hydroxy, alkoxy, Mercapto und Alkylmercapto oder Desaminorest einer natürlich vorkommenden Aminosäure oder ihrer Ester.

Croydon Printing Company Ltd.

Henkelstr. 67
4000 Düsseldorf, den 19.4.1985

Henkel KGaA
ZR-FE/Patente

Dr.Fa/KK

Patentanmeldung D 7204

"Arzneimittel mit cytostatischer Wirksamkeit, enthaltend Glycidylamine"

Es ist bekannt, daß eine Reihe alkylierender Substanzen, zu denen unter anderen die Stickstofflostderivate gehören, eine cytostatische bzw. cytotoxische Wirkung entfalten. Darüber hinaus ist bekannt, wenigstens 2 Epoxidgruppen im Molekül enthaltende Verbindungen als Cancerostatika zu verwenden. Als Beispiele für derartige Substanzen seien 4,4'-Bis(2,3-epoxypropyl)dipiperidinyl-1,1' und 1,2-15,16-Diepoxy-4,7,10,13-tetraoxohexadecan genannt. Allerdings haben die letzteren Verbindungen keine wesentliche Verbesserung in der cytostatischen Behandlung gebracht und werden daher kaum verwendet. Lediglich zur Behandlung von Hirntumoren werden sie noch hin und wieder eingesetzt.

Gegenstand der ZA 81/1530 sind Arzneimittelzubereitungen mit cytostatischer Wirksamkeit, die Di(2-hydroxy-3-brom-propyl)-substituierte Desoxy-aminopolyole enthalten. Die cytostatische Aktivität dieser Verbindungen wird auf die sich in situ bildenden Epoxidgruppen zurückgeführt.

Die vorliegende Erfindung geht von der Feststellung aus, daß Diglycidylamine mit einem aliphatischen Substituenten oder einem dritten Glycidylrest eine überraschend starke cytostatische Wirkung besitzen.

Gegenstand der Erfindung sind dementsprechend Arzneimittel mit cytostatischer Wirksamkeit, enthaltend Glycidyl-

amine der allgemeinen Formel

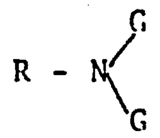

I

in der die Reste G und R eine der folgenden Bedeutungen haben:

G =

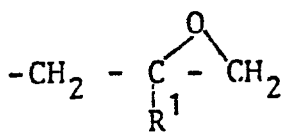

mit $R^1$ = H oder Alkyl mit 1 bis 4 Kohlenstoffatomen,

R = G oder
gesättigtes oder ungesättigtes Alkyl oder Cycloalkyl mit 1 bis 22 Kohlenstoffatomen, wobei die Reste mit einem oder mehreren der folgenden Substituenten substituiert sein können: Halogen, Hydroxy, Alkoxy, Mercapto und Alkylmercapto oder
Desaminorest einer natürlich vorkommenden Aminosäure oder ihrer Ester.

In einer bevorzugten Ausführungsform der Erfindung symbolisiert der Rest G die Glycidyl- oder die 2-Methyl-2,3-epoxypropyl-Gruppe. In einer weiteren bevorzugten Ausführungsform hat der aliphatische Rest R eine der folgenden Bedeutungen:

G oder
Alkyl mit 1 bis 10 Kohlenstoffatomen oder
Cycloalkyl mit 3 bis 10 Kohlenstoffatomen oder
Alkenyl oder Alkinyl mit 2 bis 10 Kohlenstoffatomen oder
Halogen-, Halogenhydroxy-, Dihydroxy-, Alkoxy- oder Alkoxyhydroxyalkyl mit 2 bis 6 Kohlenstoffatomen oder Desaminorest einer natürlich vorkommenden Aminosäure oder ihres Methyl- oder Ethylesters.

Besonders bevorzugte Arzneimittel mit cytostatischer Wirksamkeit enthalten Triglycidylamin und/oder 3-Chlorpropyl-diglycidylamin.

Die erfindungsgemäßen Arzneimittel können einzelne definierte Verbindungen der allgemeinen Formel I enthalten; es hat sich aber gezeigt, daß auch Wirkstoffgemische mehrerer unter die allgemeine Formel I fallender Verbindungen eine hohe cytostatische Wirkung besitzen. Der Wirkungsmechanismus der im Rahmen der Erfindung eingesetzten Glycidylamine ist im einzelnen nicht geklärt. Vermutlich sind die im Amin vorliegenden Glycidylgruppen für die cytostatische Wirksamkeit von besonderer Bedeutung.

Glycidylamine der allgemeinen Formel I mit G = Glycidyl und R = gesättigtes oder ungesättigtes Alkyl oder Cycloalkyl sind bereits bekannte Verbindungen, die beispielsweise bei der Herstellung von Kunststoff,in Brennstoffen oder als Fungizide Verwendung finden. An die Möglichkeit, solche Verbindungen für pharmazeutische Zwecke oder in Arzneimitteln zu verwenden, ist bisher noch nicht gedacht worden.

Gegenstand der Erfindung ist somit auch die Verwendung von Glycidylaminen der allgemeinen Formel

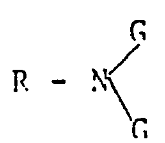

I

in der die Reste G und R eine der folgenden Bedeutungen haben:

...

G =

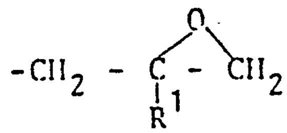

mit $R^1$ = H oder Alkyl mit 1 bis 4 Kohlenstoffatomen,
R = G oder
gesättigtes oder ungesättigtes Alkyl oder Cycloalkyl mit 1 bis 22 Kohlenstoffatomen, wobei die Reste mit einem oder mehreren der folgenden Substituenten substituiert sein können: Halogen, Hydroxy, Alkoxy, Mercapto und Alkylmercapto oder
Desaminorest einer natürlich vorkommenden Aminosäure oder ihrer Ester

in Arzneimitteln zur Therapie maligner Neoplasmen. Es können einzelne definierte Glycidylamine der allgemeinen Formel I oder Mischungen von ihnen zum Einsatz kommen.

Zur Verwendung als Cancerostatika sollten die Wirksubstanzen mittels geeigneter Vehikel appliziert werden. Hierzu eignen sich die üblichen Hilfs- bzw. Trägerstoffe für pharmakologische Zubereitungen. Im vorliegenden Fall hat sich die Verwendung wäßriger Systeme, gegebenenfalls zusammen mit verträglichen Glykolethern, wie Glykolmonoethylether oder Butylenglykolmethylether oder Propylenglykolmethylether,bewährt, insbesondere wenn der Wirkstoff parenteral appliziert werden soll. Bei oraler Verabreichung sind die pharmazeutisch üblichen Hilfs- bzw. Trägerstoffe anwendbar, sofern sie eine entsprechende Verträglichkeit mit den Glycidylverbindungen aufweisen.

Die erfindungsgemäß beschriebenen Glycidylverbindungen liegen in den Arzneimittelgemischen üblicherweise in Konzentrationen bis zu 20 Gew.-% - bezogen auf die Arzneimittelmischung - vor. Bevorzugt wird der Bereich zwischen 0,05 und 10 Gew.-% und besonders bevorzugt der zwischen 0,05 und 5 Gew.-%.

...

Im Tierexperiment hat sich die Verwendung von frisch hergestellten wäßrigen Lösungen, die i.p. oder i.v. gegeben werden, als zweckmäßig erwiesen. Einzelgaben bis zu 10 mg/kg haben sich besonders bewährt.

Arzneimittel, enthaltend Glycidylamine der allgemeinen Formel I sind wirksam gegen verschiedene Leukämieformen sowie maligne Neoplasmen, wie Lungencarcinom, Coloncarcinom, Melanome, Ependymoblastom und Sarcome.

Gegenstand der vorliegenden Erfindung sind weiterhin Glycidylamine der allgemeinen Formeln

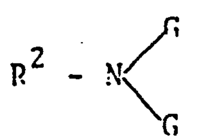

II

und

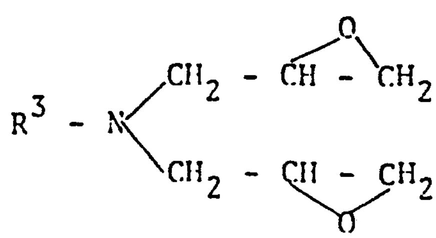

III

in der G, $R^2$ und $R^3$ eine der folgenden Bedeutungen haben:

G =

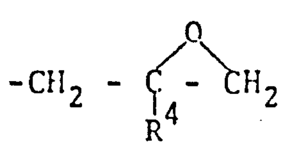

mit $R^4$ = Alkyl mit 1 bis 4 Kohlenstoffatomen,
$R^2$ = G oder
gesättigtes oder ungesättigtes Alkyl oder Cycloalkyl mit 1 bis 22 Kohlenstoffatomen, wobei die Reste mit einem oder mehreren der folgenden Substituenten substituiert sein können: Halogen, Hydroxy, Alkoxy, Mercapto und Alkylmercapto oder
Desaminorest einer natürlich vorkommenden Aminosäure oder ihrer Ester,
$R^3$ = gesättigtes oder ungesättigtes Alkyl mit 1 bis 22 Kohlenstoffatomen, wobei die Reste mit einem oder mehreren der folgenden Substituenten substituiert sind: Halogen, Hydroxy, Alkoxy, Mercapto und Alkylmercapto oder Desaminorest einer natürlich vorkommenden Aminosäure oder ihrer Ester.

Bevorzugt werden Diglycidylamine der allgemeinen Formel II, in denen G für die 2-Methyl-2,3-epoxypropyl-gruppe steht. In weiteren bevorzugten Diglycidylaminen haben die Reste $R^2$ und $R^3$ der allgemeinen Formeln II und III eine der folgenden Bedeutungen:

$R^2$ = G oder
Alkyl mit 1 bis 10 Kohlenstoffatomen oder
Cycloalkyl mit 3 bis 10 Kohlenstoffatomen oder
Alkenyl oder Alkinyl mit 2 bis 10 Kohlenstoffatomen oder
Halogen-, Halogenhydroxy-, Dihydroxy-, Alkoxy- oder Alkoxyhydroxyalkyl mit 2 bis 6 Kohlenstoffatomen oder
Desaminorest einer natürlich vorkommenden Aminosäure oder ihres Methyl- oder Ethylesters,

$R^3$ = Halogen-, Halogenhydroxy-, Dihydroxy-, Alkoxy- oder Alkoxyhydroxyalkyl mit 2 bis 6 Kohlenstoffatomen oder
Desaminorest einer natürlich vorkommenden Aminosäure oder ihres Methyl- oder Ethylesters.

Ein besonders bevorzugtes Diglycidylamin ist 3-Chlorpropyl-diglycidylamin.

Die Herstellung der Diglycidylamine erfolgt in an sich bekannter Weise durch Zutropfen des Epichlorhydrins zu dem primären Amin bei 20 bis 30 °C, vorzugsweise bei

...

25 bis 30 °C. Die Reaktion kann in einem Überschuß an Epichlorhydrin als Lösungsmittel oder in Gegenwart eines niederen Alkohols, wie zum Beispiel Methanol, Ethanol, Propanol, Isopropanol, Butanol, Isobutanol oder tertiär Butanol, durchgeführt werden. Die bevorzugte Reaktionszeit beträgt 0,5 bis 10 Stunden,insbesondere 1 bis 5 Stunden. Die anschließende Dehydrohalogenierung der intermediär entstandenen Halohydrine kann durch Zugabe von festem, gepulvertem Alkali,vorzugsweise NaOH, oder von hochkonzentrierter, wäßriger Alkalilauge bei 20 bis 25 °C vorgenommen werden.

Die Prozentangaben der nachfolgenden Beispiele beziehen sich jeweils auf Gew.-%, sofern im Einzelfall keine anderen Angaben gemacht werden.

Beispiele

Beispiel 1

Zu 187 g (2 Mol) 3-Chlorpropylamin in 200 ml Isopropanol wurden bei 27 °C innerhalb von einer Stunde 370 g (4 Mol) Epichlorhydrin getropft. Nachdem das Reaktionsgemisch 3 Stunden bei 27 °C gerührt wurde, wurden unter Kühlung bei 20 °C 180 g(4,5 Mol)Natriumhydroxid in 400 ml Wasser zugegeben, eine Stunde weitergerührt und je 200 ml Diethylether und Wasser zugesetzt. Nach Abtrennen der organischen Phase wurde die wäßrige Phase noch 3-mal mit je 200 ml Diethylether extrahiert. Nach dem Trocknen der vereinigten organischen Phasen mit Natriumsulfat, Eindampfen, Extrahieren des Rückstandes mit 4 l n-Hexan und erneutem Eindampfen wurde der Rückstand an Kieselgel (Merck) säulenchromatografiert (Eluiermittel: Methylenchlorid + 1 % Methanol).

Es wurden 300 g = 75 % 3-Chlorpropyl-diglycidylamin mit einem $R_F$-Wert: 0,57 (Kieselgel F, 10 x 20 cm, Merck; Methylenchlorid/Ethylacetat/Methanol (3 : 2 : 1)) als farblose ölige Flüssigkeit erhalten.

Analyse für $C_9H_{16}ClNO_2$ 205,7

| | | | | | | |
|---|---|---|---|---|---|---|
| Berechnet | C 52,55 | H 7,84 | N 6,85 | Cl 17,36 | O 15,56 | % |
| Gefunden | C 51,9 | H 7,51 | N 5,67 | Cl 18,9 | | % |

Epoxid-O: berechnet 15,6 gefunden 16,3 %

Das Massen-, IR- und NMR-Spektrum stützen die Struktur.

Beispiel 2

Die nachfolgenden Versuche wurden durchgeführt nach Testvorschriften des National Cancer Institute Bethesda, Maryland 200014, veröffentlicht in "Cancer Chemotherapy

Reports" Part. 3, Sept. 1972, Vol. 3, Nr. 2. Als Wirksubstanz wurde Triglycidylamin sowie 3-Chlorpropyl-diglycidylamin des vorstehenden Beispiels 1 verwendet. Die Substanz wurde als wäßrige 1 %ige Injektionslösung unmittelbar vor der Applikation frisch hergestellt.

Bei Mäusen wurde gemäß Protokoll 1200 (S. 91 c) die Tumorart P 388 (Leukämie) i.p. mit $10^6$ Zellen/Maus gesetzt. Die unbehandelten Tiere hatten eine mittlere Überlebensdauer von 10 Tagen.

Mit den behandelten Testtieren, die einer 9-tägigen i.p. Behandlung mit der in Tabelle 1 angegebenen Dosis unterworfen wurden, wurde eine signifikante Verlängerung der Lebensdauer gegenüber der mittleren Überlebensdauer der nicht mit dem Wirkstoff behandelten Tiere erzielt. Die Verlängerungstate T/C in Abhängigkeit von der Dosierung des Wirkstoffes ist in der Tabelle 1 zusammengestellt. Die Werte in Klammern bedeuten die Zahl der Tiere aus jeweils einer Gruppe von 6 die 30 Tage überlebten.

Tabelle 1: Verlängerungsrate T/C in %

| Dosis (mg/kg) / Wirkstoff | 7,50 | 3,75 | 1,87 | 0,97 | 0,48 |
|---|---|---|---|---|---|
| Triglycidylamin | - | 303 (6) | 252 | 174 | 148 |
| 3-Chlorpropyl-diglycidylamin | 303 (3) | 181 | 179 | | |

...

Patentansprüche

1. Arzneimittel mit cytostatischer Wirksamkeit, enthaltend Glycidylamine der allgemeinen Formel

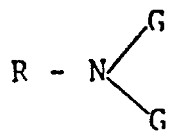

I

in der die Reste G und R eine der folgenden Bedeutungen haben:

G =

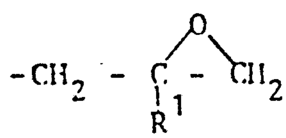

mit $R^1$ = H oder Alkyl mit 1 bis 4 Kohlenstoffatomen,
R = G oder
gesättigtes oder ungesättigtes Alkyl oder Cycloalkyl mit 1 bis 22 Kohlenstoffatomen, wobei die Reste mit einem oder mehreren der folgenden Substituenten substituiert sein können: Halogen, Hydroxy, alkoxy, Mercapto und Alkylmercapto oder Desaminorest einer natürlich vorkommenden Aminosäure oder ihrer Ester.

2. Arzneimittel nach Anspruch 1, dadurch gekennzeichnet, daß diese Glycidylamine der allgemeinen Formel I enthalten mit

G =

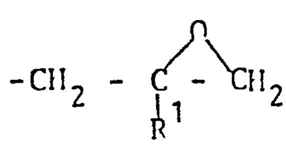

worin $R^1$ Wasserstoff oder Methyl bedeutet.

3. Arzneimittel nach Ansprüchen 1 und 2, dadurch gekennzeichnet, daß diese Glycidylamine der allgemeinen Formel I enthalten mit

Alkyl mit 1 bis 10 Kohlenstoffatomen oder Cycloalkyl mit 3 bis 10 Kohlenstoffatomen oder Alkenyl oder Alkinyl mit 2 bis 10 Kohlenstoffatomen oder
Halogen-, Halogenhydroxy-, Dihydroxy-, Alkoxy- oder Alkoxyhydroxyalkyl mit 2 bis 6 Kohlenstoffatomen oder Desaminorest einer natürlich vorkommenden Aminosäure oder ihres Methyl- oder Ethylesters.

4. Arzneimittel nach Ansprüchen 1 bis 3, dadurch gekennzeichnet, daß diese Glycidylamine der allgemeinen Formel I enthalten mit

G = 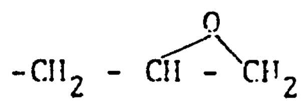 und

R = G oder 3-Chlorpropyl.

5. Verwendung von Glycidylaminen der allgemeinen Formel

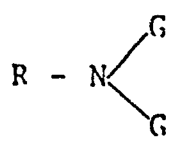 I

in der die Reste G und R eine der folgenden Bedeutungen haben:

G =

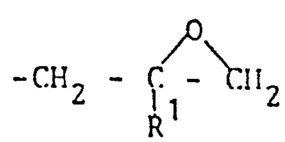

mit $R^1$ = H oder Alkyl mit 1 bis 4 Kohlenstoffatomen,
R = G oder
gesättigtes oder ungesättigtes Alkyl oder Cycloalkyl mit 1 bis 22 Kohlenstoffatomen, wobei die Reste mit einem oder mehreren der folgenden Substituenten substituiert sein können: Halogen, Hydroxy,

...

alkoxy, Mercapto und Alkylmercapto oder Desaminorest einer natürlich vorkommenden Aminosäure oder ihrer Ester zur Herstellung von Arzneimitteln zur Behandlung maligner Neoplasmen.

6. Glycidylamine der allgemeinen Formeln

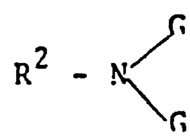

II

und

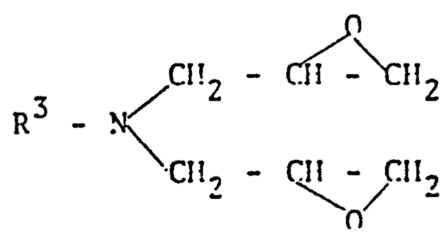

III

in der G, $R^2$ und $R^3$ eine der folgenden Bedeutungen haben:

...

G =

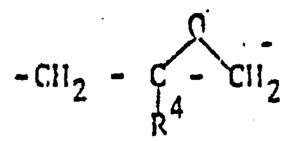

mit $R^4$ = Alkyl mit 1 bis 4 Kohlenstoffatomen,
$R^2$ = G oder
gesättigtes oder ungesättigtes Alkyl oder Cycloalkyl mit 1 bis 22 Kohlenstoffatomen,
wobei die Reste mit einem oder mehreren der folgenden Substituenten substituiert sein können:
Halogen, Hydroxy, Alkoxy, Mercapto und Alkylmercapto oder Desaminorest einer natürlich vorkommenden Aminosäure oder ihrer Ester,
$R^3$ = gesättigtes oder ungesättigtes Alkyl mit 1 bis 22 Kohlenstoffatomen, wobei die Reste mit einem oder mehreren der folgenden Substituenten substituiert sind:
Halogen , Hydroxy, Alkoxy, Mercapto und Alkylmercapto oder
Desaminorest einer natürlich vorkommenden Aminosäure oder ihre Ester

7. Glycidylamine nach Anspruch 6, dadurch gekennzeichnet, daß der Rest G in der allgemeinen Formel II

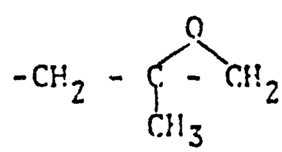

bedeutet.

8. Glycidylamine nach Ansprüchen 6 und 7, dadurch gekennzeichnet, daß der Rest $R^2$ in der allgemeinen Formel II eine der folgenden Bedeutungen haben:
G oder
Alkyl mit 1 bis 10 Kohlenstoffatomen oder Cycloalkyl mit 3 bis 10 Kohlenstoffatomen oder
Alkenyl oder Alkinyl mit 2 bis 10 Kohlenstoffatomen oder ...

Halogen-, Halogenhydroxy-, Dihydroxy-, Alkoxy- oder Alkoxyhydroxyalkyl mit 2 bis 6 Kohlenstoffatomen oder Desaminorest einer natürlich vorkommenden Aminosäure oder ihres Methyl- oder Ethylesters.

9. Glycidylamine nach Anspruch 6, dadurch gekennzeichnet, daß der Rest $R^3$ in der allgemeinen Formel III eine der folgenden Bedeutungen hat:
Halogen-, Halogenhydroxy-, Dihydroxy-, Alkoxy- oder Alkoxyhydroxyalkyl mit 2 bis 6 Kohlenstoffatomen oder Desaminorest einer natürlich vorkommenden Aminosäure oder ihres Methyl- oder Ethylesters.

10. Glycidylamine nach Ansprüchen 6 und 9, dadurch gekennzeichnet, daß der Rest $R^3$ in der allgemeinen Formel III 3-Chlorpropyl bedeutet.